# EUROPEAN PATENT SPECIFICATION

(11) **EP 3 932 477 B1**
(45) Date of publication and mention of the grant of the patent: **25.12.2024**
(21) Application number: 19917005.1
(22) Date of filing: 27.02.2019
(51) Int. Cl.: A61N 1/39, A61N 1/04, A61N 1/08

(54) **DEFIBRILLATOR AND ELECTRODE PLATES**
DEFIBRILLATOR UND ELEKTRODENPLATTEN
DÉFIBRILLATEUR ET PLAQUES D'ÉLECTRODE

(43) Date of publication of application: 05.01.2022
(73) Proprietor: Shenzhen Mindray Bio-Medical Electronics Co., Ltd., Shenzhen, Guangdong 518057 (CN)
(72) Inventor: WANG, Qi, Shenzhen, Guangdong 518057 (CN); CHEN, Dabing, Shenzhen, Guangdong 518057 (CN); DU, Run, Shenzhen, Guangdong 518057 (CN)
(74) Representative: KIPA AB
(86) International application number: PCT/CN2019/076325
(87) International publication number: WO 2020/172820

(56) References cited:
- CN-A- 103 182 147
- CN-A- 104 274 906
- CN-U- 203 154 598
- KR-B1- 101 743 414
- US-A- 5 697 955
- US-A- 5 697 955
- US-A1- 2012 299 607
- US-A1- 2014 228 903
- US-B2- 6 950 710

## Description

### TECHNICAL FIELD

The present application relates to the technical field of medical instruments, and in particular to a defibrillator and an electrode pad.

### BACKGROUND

Heart diseases such as cardiac arrests are one of the main causes of human death. About 85% to 90% of the patients with cardiac arrests undergo ventricular fibrillation in an early stage. The main way to treat ventricular fibrillation is to use a defibrillator to perform electrical defibrillation on patients. The defibrillator usually comprises a main machine and two electrode pads inserted into the main machine. The electrode pads are configured to be stuck to a target patient. The main machine generates a high voltage required for defibrillation, and transmits the high voltage required for defibrillation to the body of the target patient through the electrode pads. Whether a discharging loop composed of the main machine, the electrode pads, and the human body is normal will directly determine whether the high voltage required for defibrillation can be applied to the body of the patient normally. However, in the case of non-clinical use, the electrode pads are sealed and packaged, and are not connected to the patient, and there is no external direct connection path. Therefore, an effective loop cannot be formed for the self-check of the discharging loop of the electrode pads, making it inconvenient for device maintenance personnel of the defibrillator to maintain the defibrillator.

US2012/299607A1, KR101743414B1 and US5697955A discloses defibrillators comprising a detector configured to detect connection between electrode pads and the defibrillator by providing a conductive path between the two electrode pads, which is similar to detection of patient impedence.

### SUMMARY

The present invention provides a defibrillator according to claim 1 and a set of electrode pads according to claim 14.

Compared with the prior art, the defibrillator provided in the present application can detect whether the first electrode pad and the second electrode pad are successfully connected to a main machine, and detect whether the first electrode pad and the second electrode pad are in a normal state, making it convenient for device maintenance personnel of the defibrillator to maintain the defibrillator.

### BRIEF DESCRIPTION OF THE DRAWINGS

In order to illustrate the structural features and effects of the disclosure more clearly, they will be described in detail below with reference to the accompanying drawings and specific embodiments. Obviously, the drawings in the following description are some of the embodiments of the disclosure, and those of ordinary skill in the art would also have been able to obtain other drawings according to these drawings without involving any inventive effort.
FIG. 1 is a schematic diagram of a circuit structure of a defibrillator according to a first embodiment of the present application.
FIG. 2 is a schematic diagram of an operating environment of the defibrillator according to the first embodiment of the present application.
FIG. 3 is a schematic structural diagram of a defibrillator according to a second embodiment of the present application.
FIG. 4 is a schematic diagram of a circuit structure of a detector in the defibrillator according to the first embodiment of the present application.
FIG. 5 is a schematic diagram of an equivalent circuit of the defibrillator according to the first embodiment of the present application.
FIG. 6 is a schematic diagram of a circuit structure of the defibrillator according to the second embodiment of the present application.
FIG. 7 is a schematic structural diagram of a defibrillator, according to a third embodiment of the present application, in which a first electrode pad and a second electrode pad are not inserted into a main machine.
FIG. 8 is a schematic structural diagram of the defibrillator, according to the third embodiment of the present application, in which the first electrode pad and the second electrode pad are inserted into the main machine.
FIG. 9 is a schematic structural diagram of a defibrillator, according to a fourth embodiment of the present application, in which a first electrode pad and a second electrode pad are not inserted into a main machine.
FIG. 10 is a schematic structural diagram of the defibrillator, according to the fourth embodiment of the present application, in which the first electrode pad and the second electrode pad are inserted into the main machine.
FIG. 11 is a schematic diagram of a circuit structure of an electrode pad according to the first embodiment of the present application.
FIG. 12 is a schematic diagram of a circuit structure of the detector according to the first embodiment of the present application.
FIG. 13 is a schematic diagram of a circuit structure of a detector according to the second embodiment of the present application.
FIG. 14 is a schematic diagram of a circuit structure of a detector according to the third embodiment of the present application.
FIG. 15 is a schematic diagram of a circuit structure of a detector according to the fourth embodiment of the present application.

### DESCRIPTION OF THE PREFERRED EMBODIMENTS

The technical solutions in the embodiments of the disclosure will be clearly and completely described below in conjunction with the accompanying drawings in the embodiments of the disclosure. Obviously, the described embodiments are merely some rather than all of the embodiments of the disclosure. Based on the embodiments in the disclosure, all other embodiments that would be obtained by those of ordinary skill in the art without involving any inventive effort shall all fall within the scope of protection of the disclosure.

"Embodiment" mentioned herein means that a specific feature, structure, or characteristic described in conjunction with the embodiment may be included in at least one embodiment of the disclosure. The phrase at various locations in the specification does not necessarily refer to the same embodiment, or an independent or alternative embodiment exclusive of other embodiments. Those skilled in the art understand, in explicit and implicit manners, that an embodiment described herein may be combined with another embodiment.

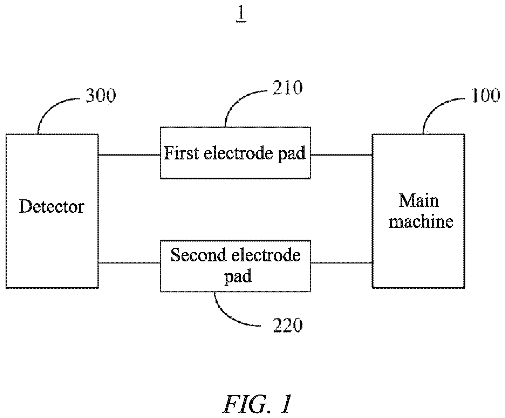

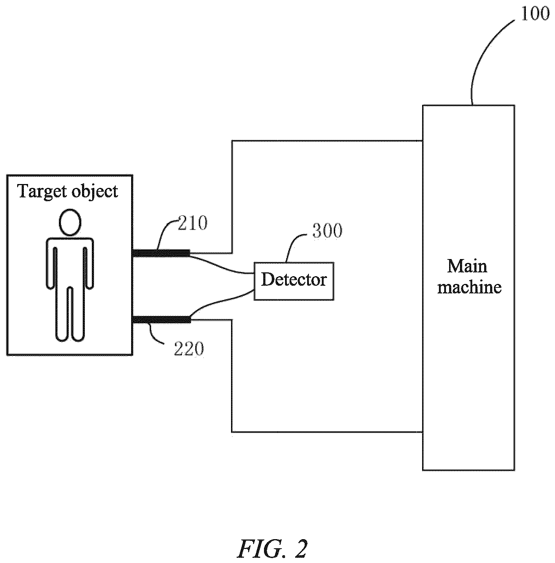

In order to make the technical solutions provided in the embodiments of the disclosure clearer, the above solutions are described in detail below with reference to the accompanying drawings. Referring to FIGS. 1 and 2 together, FIG. 1 is a schematic diagram of a circuit structure of a defibrillator according to a first embodiment of the present application; and FIG. 2 is a schematic diagram of an operating environment of the defibrillator according to the first embodiment of the present application. A defibrillator 1 comprises a main machine 100, a first electrode pad 210, a second electrode pad 220, and a detector 300. The detector 300 is electrically connected to the first electrode pad 210 and the second electrode pad 220, and is configured to detect whether the first electrode pad 210 and the second electrode pad 220 are successfully connected to the main machine 100, and detect whether the first electrode pad 210 and the second electrode pad 220 are normal. The detector 300 may be arranged in the main machine 100 or outside the main machine 100. In the figure, it is shown that the detector 300 is arranged outside the main machine 100.

Compared with the prior art, the defibrillator provided in the present application can detect whether the first electrode pad and the second electrode pad are successfully connected, and detect whether the first electrode pad and the second electrode pad are in a normal state, making it convenient for device maintenance personnel of the defibrillator to maintain the defibrillator.

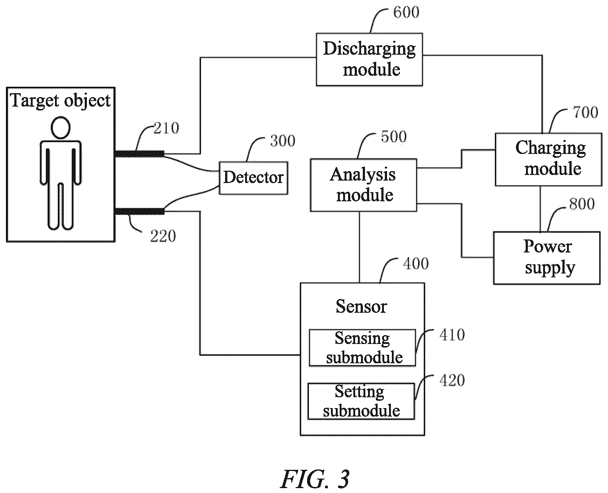

Referring to FIG. 3, FIG. 3 is a schematic structural diagram of a defibrillator according to a second embodiment of the present application. In this embodiment, the defibrillator 1 further comprises a sensor 400, an analysis module 500, a discharging module 600, a charging module 700, and a power supply 800. The sensor 400, the analysis module 500, the discharging module 600, the charging module 700, and the power supply 800 may be arranged in the main machine 100 or outside the main machine 100. When the defibrillator 1 is in use, the first electrode pad 210 and the second electrode pad 220 are stuck to a target object. For example, the first electrode pad 210 and the second electrode pad 220 are stuck to but not limited to being stuck to the chest of the target object. The sensor 400 is electrically connected to the first electrode pad 210 and the second electrode pad 220, and the sensor 400 senses the heart activity of the target object through the first electrode pad 210 and the second electrode pad 220 to obtain a corresponding electrocardiogram (ECG) signal. The analysis module 500 may analyze the electrocardiogram signal to determine whether the target object meets an electric shock condition. For example, when it is determined according to the ECG signal that a heart rhythm of the target object is characterized by at least one of ventricular fibrillation, ventricular tachycardia, and ventricular flutter, it may be determined that the target object meets the electric shock condition. When it is determined according to the ECG signal that the heart rhythm of the target object is characterized by any one of bradycardia, electromechanical dissociation, idioventricular rhythm, and normal heart rhythm, it may be determined that the target object does not meet the electric shock condition. When the target object meets the electric shock condition and an electric shock instruction is received, the discharging module 600 can release defibrillation energy through the first electrode pad 210 and the second electrode pad 220 to treat the target object. In an embodiment, when the target object meets the electric shock condition, an electric shock instruction is automatically triggered. In another embodiment, the defibrillator 1 comprises a discharging button, and when the discharging button is pressed, an electric shock instruction is triggered. Specifically, when the target object meets the electric shock condition, an alarm unit of the defibrillator 1 issues prompt information, which is used to give a prompt that the target object is allowed to be subjected to an electric shock, and an operator can press the discharging button according to the prompt information, so as to trigger the electric shock instruction.

Still further, the sensor 400 comprises a sensing submodule 410 and a setting submodule 420. When the first electrode pad 210 and the second electrode pad 220 are stuck to the target object, the sensing submodule is configured to sense a first signal when the first electrode pad 210 and the second electrode pad 220 are stuck to the target object, and the analysis module 500 is configured to analyze whether there is a second signal that represents a pacemaker in the first signal. The setting submodule 420 is configured to subtract, when there is a second signal that represents a pacemaker in the first signal, the second signal from the first signal to obtain the ECG signal; and the setting submodule 420 is further configured to set the first signal as the ECG signal when there is no second signal that represents a pacemaker in the first signal. In this implementation, whether the target object wears a pacemaker is detected to prevent interference to the ECG signal when the target object wears a pacemaker.

It can be understood that the term "module" used in the present application may be an integrated chip with a certain function, or a common circuit composed of circuit components, or in other forms. The specific form of implementing the "module" is not limited in the present application, provided that the corresponding function can be implemented. For example, the function of the "analysis module 500" mentioned above is to analyze the electrocardiogram signal to determine whether the target object meets the electric shock condition. The specific form of implementing the "analysis module 500" may be an integrated chip or a common circuit composed of circuit components.

The charging module 700 is configured to receive and store electric energy, and when the target object meets the electric shock condition and an electric shock instruction is received, the energy stored in the charging module 700 is loaded onto the first electrode pad 210 and the second electrode pad 220 by means of the discharging module 600 and transferred to the target object. In an embodiment, the charging module 700 and the discharging module 600 may also be integrated into a charging and discharging module. The charging and discharging module can not only receive a charging signal and store energy, but also release the stored energy. In an embodiment, the power supply 800 may be a primary battery or a rechargeable battery.

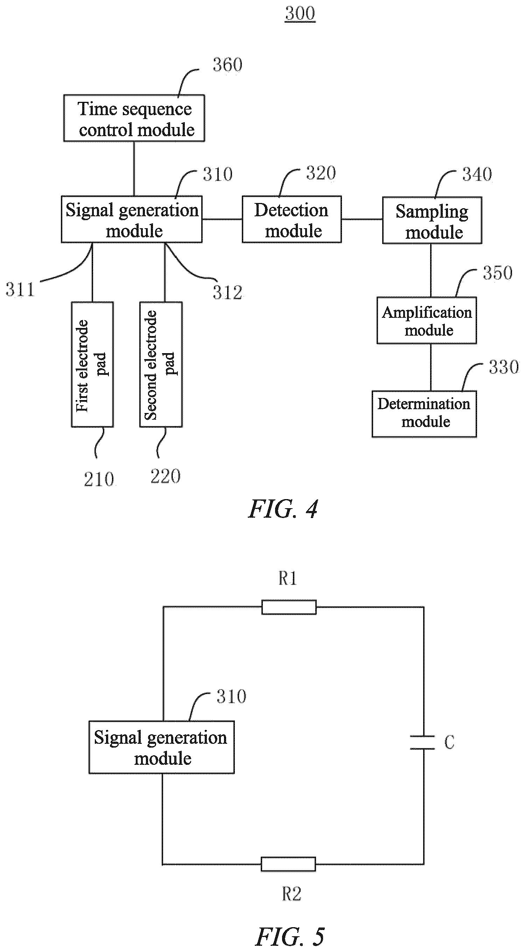

Referring also to FIG. 4, FIG. 4 is a schematic diagram of a circuit structure of a detector in the defibrillator according to the first embodiment of the present application. The detector 300 comprises a signal generation module 310, a detection module 320, and a determination module 330. The signal generation module 310 is configured to generate a test signal, the detection module 320 is configured to detect a detection signal generated after the test signal passes through the first electrode pad 210 and the second electrode pad 220, and the determination module 330 determines, according to the detection signal, whether the first electrode pad 210 and the second electrode pad 220 are successfully connected to the main machine 100 and determines whether the first electrode pad 210 and the second electrode pad 220 are normal.

Specifically, when the detection module 320 receives the detection signal, the determination module 330 determines that the first electrode pad 210 and the second electrode pad 220 are successfully connected to the main machine 100. When the detection module 300 fails to receive the detection signal, the determination module 330 determines that the first electrode pad 210 and the second electrode pad 220 are not successfully connected to the main machine 100.

Further, the determination module 330 determines, according to magnitude of a resistance value in the detection signal received by the detection module 320, whether the first electrode pad 210 and the second electrode pad 220 are normal. For example, when a conductive adhesive in each of the first electrode pad 210 and the second electrode pad 220 has poor performance (for example, the conductive adhesive is dry), the resistance value in the obtained detection signal exceeds a preset resistance value; and when the conductive adhesive in each of the first electrode pad 210 and the second electrode pad 220 has good performance, the resistance value in the obtained detection signal is less than or equal to the preset resistance value.

In this embodiment, the detection module 320 can determine, according to whether the detection signal can be received, whether the first electrode pad 210 and the second electrode pad 220 are successfully connected to the main machine 100, and at the same time, can determine, according to the magnitude of the resistance value in the detection signal, whether the first electrode pad 210 and the second electrode pad 220 are normal, so as to achieve the technical effect of performing a plurality of determinations in one measurement.

Further, the signal generation module 310 comprises a first terminal 311 and a second terminal 312. The first terminal 311 is electrically connected to the first electrode pad 210, the second terminal 312 is electrically connected to the second electrode pad 220, and the test signal is output to the first electrode pad 210 and the second electrode pad 220 via the first terminal 311 and the second terminal 312.

Further, the detection signal is an analogue signal, the detector 300 comprises a sampling module 340, and the sampling module 340 is configured to sample the detection signal; and the determination module 330 determines, according to the sampled detection signal, whether the first electrode pad 210 and the second electrode pad 220 are successfully connected to the main machine 100 and determines whether the first electrode pad 210 and the second electrode pad 220 are normal, wherein the sampled detection signal is a digital signal. The test signal is an analogue signal, and the sampling module 340 samples the test signal to obtain a digital signal, so as to reduce the amount of data processed when the determination module 330 performs a determination.

Further, the detector 300 further comprises an amplification module 350, and the amplification module 350 is configured to amplify the detection signal and then output the signal to the sampling module 340. In this case, the sampling module 340 is configured to sample the amplified detection signal. After the amplification module 350 amplifies the detection signal, the accuracy of a result of determination can be improved.

Referring to FIG. 5, FIG. 5 is a schematic diagram of an equivalent circuit of the defibrillator according to the first embodiment of the present application. A contact resistance between the first electrode pad 210 and the main machine 100 is equivalent to that of a first resistor R1, a contact resistance between the second electrode pad 220 and the main machine 100 is equivalent to that of a second resistor R2, a capacitance between the first electrode pad 210 and the second electrode pad 220 is equivalent to that of a capacitor C, one terminal of the capacitor is connected to the first resistor R1, and the other terminal of the capacitor C is connected to the second resistor R2. That is, when the first electrode pad 210 and the second electrode pad 220 are both connected to the main machine 100 well, the signal generation module 310, the first resistor R1, the second resistor R2, and the capacitor C form a loop. After a test signal with a certain frequency and amplitude that is generated by the signal generation module 310 passes through the first electrode pad 210 and the second electrode pad 220, the detection module 320 detects the signal on the capacitor to obtain the detection signal. According to the detection signal, the determination module 330 determines whether the first electrode pad 210 is successfully connected to the main machine 100, determines whether the second electrode pad 220 is successfully connected to the main machine 100, and determines whether the first electrode pad 210 and the second electrode pad 220 are normal. The specific determination process is described in detail as follows.

When the first electrode pad 210 is normal and the first electrode pad 210 is successfully connected to the main machine 100, a resistance value of the first resistor R1 is very small; and when a second electrode is normal and the second electrode pad 220 is successfully connected to the main machine 100, a resistance value of the second resistor R2 is very small. When the first electrode pad 210 is normal and the first electrode pad 210 is successfully connected to the main machine 100, the resistance value of the first resistor R1 is generally less than 0.2 ohm; and when a second electrode is normal and the second electrode pad 220 is successfully connected to the main machine 100, the resistance value of the second resistor R2 is generally less than 0.2 ohm. An equivalent capacitance between the first electrode pad 210 and the second electrode pad is generally between 100 pF and 200 pF. When the first electrode pad 210 is normal and the first electrode pad 210 is successfully connected to the main machine 100, and when the second electrode pad 220 is normal and the second electrode pad 220 is successfully connected to the main machine 100, after a test signal with a certain frequency and amplitude that is generated by the signal generation module 310 passes through the first electrode pad 210 and the second electrode pad 220, the detection module 320 can obtain a corresponding detection signal according to the test signal.

When the above loop is abnormal, for example, the first electrode pad 210 is not connected to the main machine 100, or the second electrode pad 220 is not connected to the main machine 100, and when the test signal is loaded onto the first electrode pad 210 and the second electrode pad 220, the detection module 320 can hardly collect detection signals. Similarly, when the first electrode pad 210 and the second electrode pad 220 do not have opposite areas, or when the conductive adhesive in the first electrode pad 210 is not sufficiently viscous, or the conductive adhesive in the first electrode pad 210 is dry, or the conductive adhesive in the second electrode pad 220 is not sufficiently viscous, or the conductive adhesive in the second electrode pad 220 is dry, no capacitance can be formed between the first electrode pad 210 and the second electrode pad 220. In this case, a voltage divider network of the first resistor R1, the second resistor R2, and the capacitor cannot be formed, and then an impedance value obtained according to the detection signal is greater than a preset impedance value.

Further, the test signal comprises a first test sub-signal and a second test sub-signal. The detection signal comprises a first detection sub-signal and a second detection sub-signal; when the first test sub-signal is loaded onto the first electrode pad 210 and the second electrode pad 220, a corresponding detection signal is the first detection sub-signal, and when the second test sub-signal is loaded onto the first electrode pad 210 and the second electrode pad 220, a corresponding detection signal is the second detection sub-signal; and the determination module 330 determines, according to a difference between the first detection sub-signal and the second detection sub-signal, whether the first electrode pad 210 and the second electrode pad 220 are successfully connected to the main machine 100, and determines whether the first electrode pad 210 and the second electrode pad 220 are normal. In this embodiment, the accuracy of a result of determination can be improved by comparing the first detection sub-signal and the second detection sub-signal.

Specifically, the signal generation module 310 generates a first test sub-signal and a second test sub-signal. When the first test sub-signal is loaded onto the first electrode pad 210 and the second electrode pad 220, the detection signal obtained by the detection module 320 is called a first detection sub-signal; and when the second test sub-signal is loaded onto the first electrode pad 210 and the second electrode pad 220, the detection signal obtained by the detection module 320 is called a second detection sub-signal. In this embodiment, the accuracy of the result of determination can be improved by using the two detection results.

Further, the detector 300 also comprises a time sequence control module 360. The time sequence control module 360 controls the first test sub-signal to be loaded onto the first electrode pad 210 and the second electrode pad 220 before the second test sub-signal, and a frequency of the second test sub-signal is different from that of the first test sub-signal, or an amplitude value of the second test sub-signal is greater than that of the first test sub-signal. In this embodiment, the time sequence control module 360 is electrically connected to the signal generation module 310, so as to control the time sequence of loading the first test sub-signal and the second test sub-signal generated by the signal generation module 310 onto the first electrode pad 210 and the second electrode pad 220.

A frequency characteristic of a capacitor is Xc = 1/(2πfc), where Xc is an equivalent impedance of the capacitor, and fc is a frequency of a detection signal. Therefore, for a test signal with a different frequency, a corresponding equivalent impedance of the capacitor is different. When the first electrode pad 210 and the second electrode pad 220 cannot face each other normally due to packaging and other causes, coupling between the first electrode pad 210 and the second electrode pad 220 is relatively poor, and when a frequency of the detection signal is a low frequency, it cannot be accurately determined whether the first electrode pad 210 and the second electrode pad 220 are successfully connected to the main machine 100, and it cannot be accurately determined whether the first electrode pad 210 and the second electrode pad 220 are normal. Therefore, the determination module 330 can improve the accuracy of the result of determination by comparing a first detection sub-signal and a second detection sub-signal corresponding to a first test sub-signal and a second test sub-signal with different frequencies. Further, the amplitude of the second test sub-signal is greater than the amplitude value of the first test sub-signal, which is beneficial to improving a signal-to-noise ratio and further improves the accuracy of the result of determination.

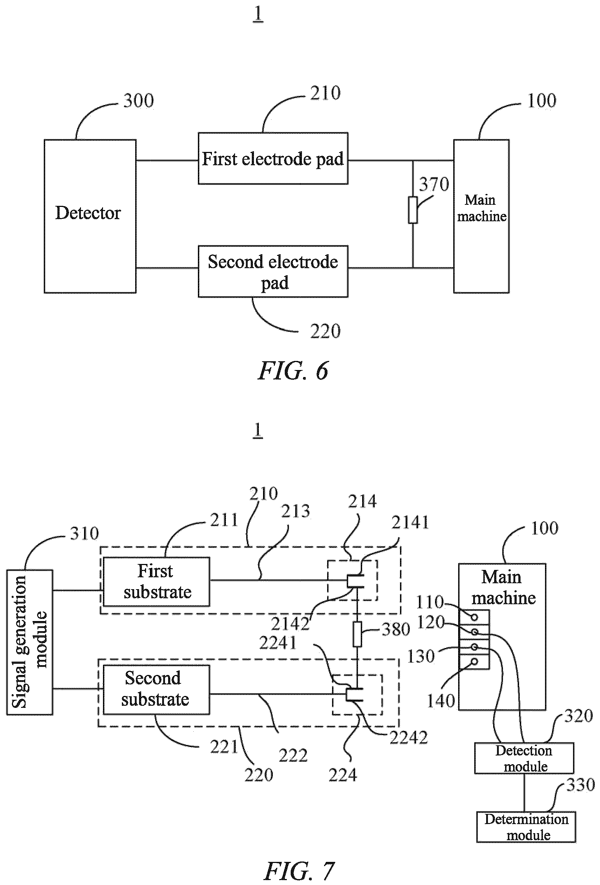

Further, referring to FIG. 6, FIG. 6 is a schematic diagram of a circuit structure of the defibrillator according to the second embodiment of the present application. A structure of the defibrillator 1 provided in this implementation is basically the same as that of the defibrillator 1 provided in the first implementation of the present application, except that in this implementation, the detector 300 further comprises a first test resistor 370. One terminal of the first test resistor 370 is electrically connected to the first electrode pad 210, and the other terminal of the first test resistor 370 is electrically connected to the second electrode pad 220. One terminal of the detection module 320 is electrically connected to a node between the first test resistor 370 and the first electrode pad 210, and the other terminal of the detection module 320 is electrically connected to a node between the first test resistor 370 and the second electrode pad 220.

When a capacitance cannot be formed between the first electrode pad 210 and the second electrode pad 220 due to various causes, the first test resistor 370 is added to be electrically connected between the first electrode pad 210 and the second electrode pad 220 in this embodiment, so as to improve the certainty of forming a loop required for the test. The detection module 320 compares a detected resistance value with a resistance value of the first test resistor 370 to determine whether the first electrode pad 210 is successfully connected to the main machine 100 and determine whether the second electrode pad 220 is successfully connected to the main machine 100. Specifically, when the first electrode pad 210 is successfully connected to the main machine 100 and the second electrode pad 220 is successfully connected to the main machine 100, a difference between the resistance value represented by the detection signal and the resistance value of the first test resistor 370 is within a preset range; and when the first electrode pad 210 is absuccessfully connected to the main machine 100 or the second electrode pad 220 is not successfully connected to the main machine 100 or the first electrode pad 210 and the second electrode pad 220 are not successfully connected to the main machine 100, the difference between the resistance value represented by the detection signal and the resistance value of the first test resistor 370 is beyond the preset range. Therefore, in this embodiment, by detecting whether the first test resistor 370 is connected normally, it can be determined whether the first electrode pad 210 is successfully connected to the main machine 100, and whether the second electrode pad 220 is successfully connected to the main machine 100.

Further, in normal use of the defibrillator 1, when a user takes out the first electrode pad 210 and the second electrode pad 220 from a packaging bag, and sticks the first electrode pad 210 and the second electrode pad 220 to different parts of the body of the target object, it is possible to disconnect the first test resistor 370 and the first electrode pad 210, or disconnect the first test resistor 370 and the second electrode pad 220, or disconnect the first test resistor 370 and both the first electrode pad 210 and the second electrode pad 220, thereby not affecting normal use of the defibrillator 1.

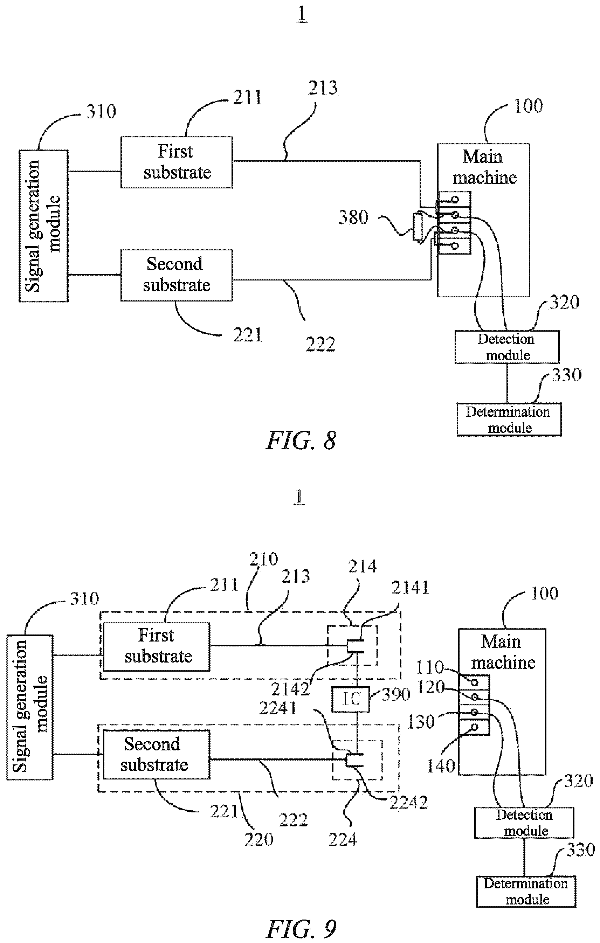

Further, referring to FIGS. 7 and 8 together, FIG. 7 is a schematic structural diagram of a defibrillator, according to a third embodiment of the present application, in which a first electrode pad and a second electrode pad are not inserted into a main machine; and FIG. 8 is a schematic structural diagram of the defibrillator, according to the third embodiment of the present application, in which the first electrode pad and the second electrode pad are inserted into the main machine. The structure of the defibrillator 1 provided in this embodiment is basically the same as that of the defibrillator 1 provided in the first embodiment of the present application. The first electrode pad 210 and the second electrode pad 220 are inserted into the socket holes in the main machine 100 through connectors. The difference is that in this embodiment, the detector 300 further comprises a second test resistor 380. One terminal of the second test resistor 380 is electrically connected to a connector of the first electrode pad 210, the other terminal of the second test resistor 380 is electrically connected to a plug of the second electrode pad 220, the detection module 320 is electrically connected to an socket hole of the main machine 100, and the determination module 330 determines, according to a resistance value detected by the detection module 320 and a resistance value of the second test resistor 380, whether the first electrode pad 210 and the second electrode pad 220 are successfully connected to the main machine 100.

Correspondingly, the first electrode pad 210 comprises a first substrate 211, a first wire 213, and a first connector 214. The first connector 214 comprises a first pin 2141 and a second pin 2142. The second electrode pad 220 comprises a second substrate 221, a second wire 223, and a second connector 224. The second connector 224 comprises a third pin 2241 and a fourth pin 2242. The first substrate 211 is electrically connected to the first pin 2141 with the first wire 213, one terminal of the second test resistor 380 is electrically connected to the second pin 2142, the other terminal of the second test resistor 380 is electrically connected to the third pin 2241, and the second substrate 221 is electrically connected to the fourth pin 2242 with the second wire 223. The main machine 100 comprises a first socket hole 110, a second socket hole 120, a third socket hole 130, and a fourth socket hole 140. The first pin 2141 corresponds to the first socket hole 110, the second pin 2142 corresponds to the second socket hole 120, the third pin 2241 corresponds to the third socket hole 130, and the fourth pin 2242 corresponds to the fourth socket hole 140. The detection module 320 is electrically connected to the second socket hole 120 and the third socket hole 130, and the detection module 320 detects a resistance value between the second socket hole 120 and the third socket hole 130. The determination module 330 further determines, according to the resistance value detected by the detection module 320 and the resistance value of the second test resistor 380, whether the first electrode pad 210 and the second electrode pad 220 are successfully connected to the main machine 100.

It can be understood that in the embodiment, the detector 300 may further comprise a sampling module and an amplification module. For a connection relationship between other modules in the detector 300 and each of the sampling module and the amplification module and functions of the acquisition module and the amplification module, reference may be made to FIG. 4 and the related description. Details are not described herein.

In this embodiment, when the first electrode pad 210 is inserted into the main machine 100, the first pin 2141 is inserted into the first socket hole 110, and the second pin 2142 is inserted into the second socket hole 120. When the second electrode pad 220 is inserted into the main machine 100, the third pin 2241 is inserted into the third socket hole 130, and the fourth pin 2242 is inserted into the fourth socket hole 140. When the first electrode pad 210 is disconnected from the main machine 100, the first pin 2141 and the second pin 2142 are disconnected from the main machine 100. When the second electrode pad 220 is disconnected from the main machine 100, the third pin 2241 and the fourth pin 2242 are disconnected from the main machine 100. Since one terminal of the second test resistor 380 is electrically connected to the second pin 2142, and the other terminal thereof is electrically connected to the third pin 2241, when the first electrode pad 210 and the second electrode pad 220 are inserted into the main machine 100, one terminal of the second test resistor 380 is electrically connected to the second socket hole 120, and the other terminal of the second test resistor 380 is electrically connected to the third socket hole 130. In this case, the detection module 320 can be electrically connected to the second test resistor 380, and the detection module 320 can detect the resistance value of the second test resistor 380. When the first electrode pad 210 and the second electrode pad 220 are both disconnected from the main machine 100, the second test resistor 380 is disconnected from the second socket hole 120 and the third socket hole 130. In this case, the detection module 320 fails to detect the resistance value of the second resistor R2. Therefore, the determination module 330 determines, according to the resistance value detected by the detection module 320 and the resistance value of the second test resistor 380, whether the first electrode pad 210 is successfully connected to the main machine 100 and determines whether the second electrode pad 220 is successfully connected to the main machine 100.

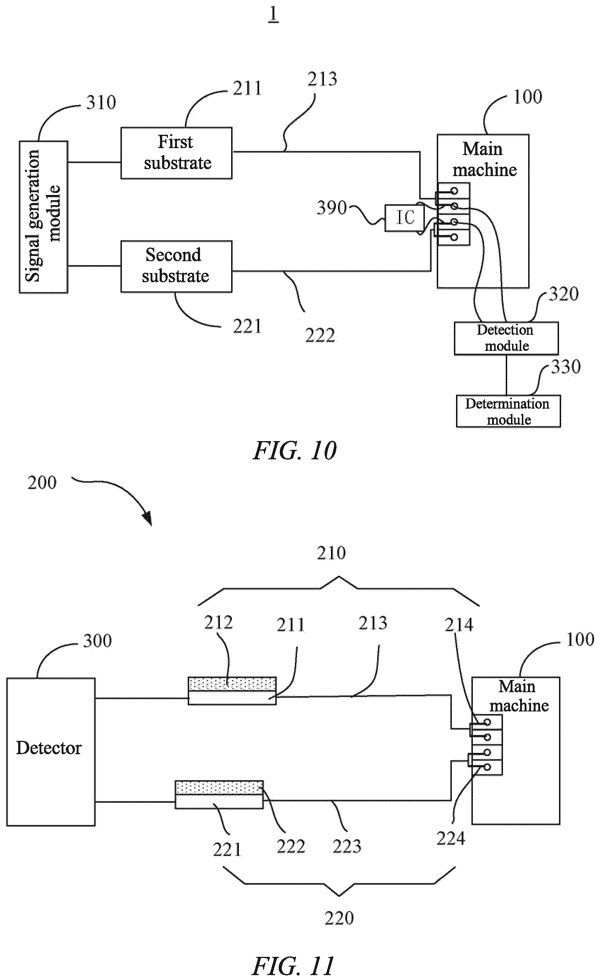

Further, referring to FIGS. 9 and 10, FIG. 9 is a schematic structural diagram of a defibrillator, according to a fourth embodiment of the present application, in which a first electrode pad and a second electrode pad are not inserted into a main machine; and FIG. 10 is a schematic structural diagram of the defibrillator, according to the fourth embodiment of the present application, in which the first electrode pad and the second electrode pad are inserted into the main machine. The structure of the defibrillator 1 provided in this embodiment is basically the same as that of the defibrillator 1 provided in the first embodiment of the present application, except that in this embodiment, the first electrode pad 210 and the second electrode pad 220 are inserted into the socket holes in the main machine 100 through plugs. The detector 300 further comprises an integrated chip 390 (represented by IC in the figure). one terminal of the integrated chip 390 is electrically connected to a plug of the first electrode pad 210, and the other terminal of the integrated chip 390 is electrically connected to a plug of the second electrode pad 220. The detection module 320 is electrically connected to an socket hole of the main machine 100, and the determination module 330 determines, according to whether the detection module 320 can read content of the integrated chip 390, whether the first electrode pad 210 and the second electrode pad 220 are successfully connected to the main machine 100.

Specifically, the first electrode pad 210 comprises a first substrate 211, a first wire 213, and a first connector 214. The first connector 214 comprises a first pin 2141 and a second pin 2142. The second electrode pad 220 comprises a second substrate 221, a second wire 223, and a second connector 224. The second connector 224 comprises a third pin 2241 and a fourth pin 2242. The main machine 100 comprises a first socket hole 110, a second socket hole 120, a third socket hole 130, and a fourth socket hole 140. The first substrate 211 is electrically connected to the first pin 2141 with the first wire 213, the integrated chip 390 is separately electrically connected to the second pin 2142 and the third pin 2241, the second substrate 221 is electrically connected to the fourth pin 2242 with the second wire 223, the first pin 2141 corresponds to the first socket hole 110, the second pin 2142 corresponds to the second socket hole 120, the third pin 2241 corresponds to the third socket hole 130, and the fourth pin 2242 corresponds to the fourth socket hole 140. The detection module 320 is electrically connected to the second socket hole 120 and the third socket hole 130, and the determination module 330 determines, according to whether the detection module 320 can read the content of the integrated chip 390, whether the electrode pad 210 and the second electrode pad 220 are successfully connected to the main machine 100.

In this embodiment, when the first electrode pad 210 is inserted into the main machine 100, the first pin 2141 is inserted into the first socket hole 110, and the second pin 2142 is inserted into the second socket hole 120. When the second electrode pad 220 is inserted into the main machine 100, the third pin 2241 is inserted into the third socket hole 130, and the fourth pin 2242 is inserted into the fourth socket hole 140. When the first electrode pad 210 is disconnected from the main machine 100, the first pin 2141 and the second pin 2142 are disconnected from the main machine 100. When the second electrode pad 220 is disconnected from the main machine 100, the third pin 2241 and the fourth pin 2242 are disconnected from the main machine 100. Since the integrated chip 390 is jointly electrically connected to the second pin 2142 and the third pin 2241, when the first electrode pad 210 and the second electrode pad 220 are inserted into the main machine 100, one terminal of the integrated chip 390 is electrically connected to the second socket hole 120, and the other terminal of the integrated chip 390 is electrically connected to the third socket hole 130. In this case, the detection module 320 can be electrically connected to the integrated chip 390, and the detection module 320 can detect the content of the integrated chip 390. When the first electrode pad 210 and the second electrode pad 220 are both disconnected from the main machine 100, the second test resistor 380 is disconnected from the second socket hole 120 and the third socket hole 130. In this case, the detection module 320 fails to read the content of the integrated chip 390. Therefore, when the detection module 320 can read the content of the integrated chip 390, the determination module 330 determines that the electrode pad 210 and the second electrode pad 220 are successfully connected to the main machine 100, that is, the first electrode pad 210 and the second electrode pad 220 are successfully connected to the main machine 100; and when the detection module 320 fails to read the content of the integrated chip 390, the determination module 330 determines that the first electrode pad 210 and the second electrode pad 220 are not successfully connected to the main machine 100.

Further, the integrated chip 390 stores a manufacturing date and an expiration date of the first electrode pad 210 and the second electrode pad 220, and when the detection module 320 reads the manufacturing date and the expiration date of the first electrode pad 210 and the second electrode pad 220 from the integrated chip 390, the determination module 330 further determines, according to a current date, the manufacturing date, and the expiration date, whether the first electrode pad 210 and the second electrode pad 220 have expired.

The present application further provides an electrode pad 200. The electrode pad 200 of the present application is described below in conjunction with the defibrillator 1 described above. Referring to FIG. 11, FIG. 11 is a schematic diagram of a circuit structure of an electrode pad according to the first embodiment of the present application. The electrode pad 200 comprises a first electrode pad 210, a second electrode pad 220, and a detector 300. The first electrode pad 210 comprises a first substrate 211, a first conductive adhesive 212, a first wire 213, and a first connector 214. The first conductive adhesive 212 is arranged on the first substrate 211, and the first substrate 211 is electrically connected to the first connector 214 with the first wire 213. The second electrode pad 220 comprises a second substrate 221, a second conductive adhesive 222, a second wire 223, and a second connector 224. The second conductive adhesive 222 is arranged on the second substrate 221, and the second substrate 221 is electrically connected to the second connector 224 with the second wire 223. The first connector 214 and the second connector 224 are configured to be inserted into the main machine 100. The detector 300 is electrically connected to the first substrate 211 and the second substrate 221. The detector 300 is configured to detect whether the first connector 214 and the second connector 224 are successfully connected to the main machine 100 when the first connector 214 and the second connector 224 are inserted into the main machine 100, and the detector 300 is further configured to detect whether the first electrode pad 210 and the second electrode pad 220 are normal.

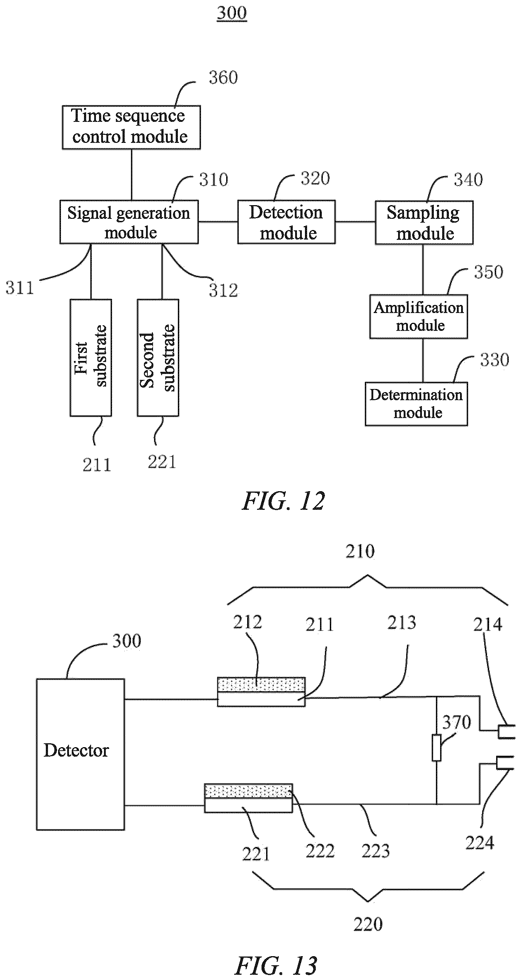

Specifically, referring also to FIG. 12, FIG. 12 is a schematic diagram of a circuit structure of the detector according to the first embodiment of the present application. The detector 300 comprises a signal generation module 310, a detection module 320, and a determination module 330. The signal generation module 310 is configured to generate a test signal, and the detection module 320 is configured to detect a detection signal generated after the test signal passes through the first substrate 211 and the second substrate 221. According to the detection signal, the determination module 330 determines whether the first connector 214 and the second connector 224 are successfully connected to the main machine 100, determines whether the first wire 213 and the second wire 223 are open-circuited, and determines whether the first conductive adhesive 212 and the second conductive adhesive 222 are normal.

When the detection module 320 receives the detection signal, the determination module 330 determines that the first electrode pad 210 and the second electrode pad 220 are successfully connected to the main machine 100, and when the detection module 320 fails to receive the detection signal, the determination module 330 determines that the first electrode pad 210 and the second electrode pad 220 are not successfully connected to the main machine 100.

The determination module 330 determines, according to magnitude of a resistance value in the detection signal received by the detection module 320, whether the first electrode pad 210 and the second electrode pad 220 are normal. For example, when a conductive adhesive in each of the first electrode pad 210 and the second electrode pad 220 has poor performance (for example, the conductive adhesive is dry), the resistance value in the obtained detection signal exceeds a preset resistance value; and when the conductive adhesive in each of the first electrode pad 210 and the second electrode pad 220 has good performance, the resistance value in the obtained detection signal is less than or equal to the preset resistance value.

In this embodiment, the detection module 320 can determine, according to whether the detection signal can be received, whether the first electrode pad 210 and the second electrode pad 220 are successfully connected to the main machine 100, and at the same time, can determine, according to the magnitude of the resistance value in the detection signal, whether the first electrode pad 210 and the second electrode pad 220 are normal, so as to achieve the technical effect of performing a plurality of determinations in one measurement.

Specifically, the signal generation module 310 comprises a first terminal 311 and a second terminal 312, the first terminal 311 is electrically connected to the first substrate 211, the second terminal 312 is electrically connected to the second substrate 221, and the test signal is output to the first substrate 211 and the second substrate 221 via the first terminal 311 and the second terminal 312.

Further, the detection signal is an analogue signal, and the detector 300 comprises a sampling module 340. The sampling module 340 is configured to sample the detection signal; and the determination module 330 determines, according to the sampled detection signal, whether the first connector 214 and the second connector 224 are successfully connected to the main machine 100 and determines whether the first electrode pad 210 and the second electrode pad 220 are normal, wherein the sampled detection signal is a digital signal.

Further, the detector 300 further comprises an amplification module 350, and the amplification module 350 is configured to amplify the detection signal and then output the signal to the sampling module 340. In this case, the sampling module is configured to sample the amplified detection signal.

Further, the test signal comprises a first test sub-signal and a second test sub-signal. The detection signal comprises a first detection sub-signal and a second detection sub-signal. When the first test sub-signal is loaded onto the first substrate 211 and the second substrate 221, a corresponding detection signal is the first detection sub-signal; and when the second test sub-signal is loaded onto the first substrate 211 and the second substrate 221, a corresponding detection signal is the second detection sub-signal. The determination module 330 determines, according to a difference between the first detection sub-signal and the second detection sub-signal, whether the first connector 214 and the second connector 224 are successfully connected to the main machine 100 and determines whether the first electrode pad 210 and the second electrode pad 220 are normal.

Further, the detector 300 also comprises a time sequence control module 360. The time sequence control module 360 controls the first test sub-signal to be loaded onto the first substrate 211 and the second substrate 221 before the second test sub-signal, and a frequency of the second test sub-signal is different from that of the first test sub-signal, or an amplitude value of the second test sub-signal is greater than that of the first test sub-signal.

Referring to FIG. 13, FIG. 13 is a schematic diagram of a circuit structure of a detector according to the second embodiment of the present application. The detector 300 provided in this embodiment is basically the same as the detector 300 provided in the first embodiment, except that in this embodiment, the detector 300 further comprises a first test resistor 370. One terminal of the first test resistor 370 is electrically connected to the first substrate 211, the other terminal of the first test resistor 370 is electrically connected to the second substrate 221, one terminal of the detection module 320 is electrically connected to a node between the first test resistor 370 and the first substrate 211, and the other terminal of the detection module 320 is electrically connected to a node between the first test resistor 370 and the second substrate 221.

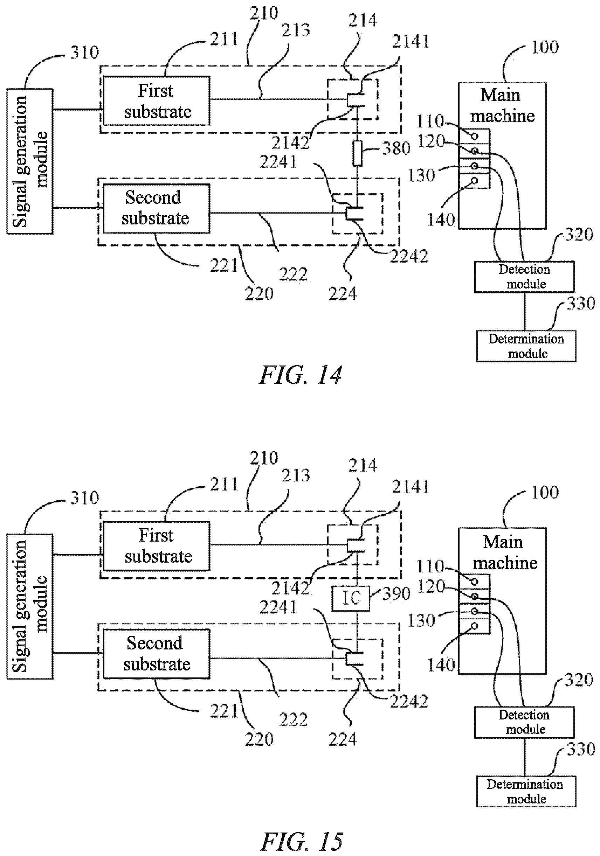

Referring to FIG. 14, FIG. 14 is a schematic diagram of a circuit structure of a detector according to the third embodiment of the present application. The detector 300 provided in this embodiment is basically the same as the detector 300 provided in the first embodiment, except that in this embodiment, the detector 300 further comprises a second test resistor 380. One terminal of the second test resistor 380 is electrically connected to the first connector 214, the other terminal of the second test resistor 380 is electrically connected to the second connector 224, the detection module 320 is electrically connected to an socket hole of the main machine 100, and the determination module 330 determines, according to a resistance value detected by the detection module 320 and a resistance value of the second test resistor 380, whether the first electrode pad 210 and the second electrode pad 220 are successfully connected to the main machine 100.

The first connector 214 comprises a first pin 2141 and a second pin 2142, and the second connector 224 comprises a third pin 2241 and a fourth pin 2242. The main machine 100 comprises a first socket hole 110, a second socket hole 120, a third socket hole 130, and a fourth socket hole 140. The first substrate 211 is electrically connected to the first pin 2141 with the first wire 213, one terminal of the second test resistor 380 is electrically connected to the second pin 2142, the other terminal of the second test resistor 380 is electrically connected to the third pin 2241, and the second substrate 221 is electrically connected to the fourth pin 2242 with the second wire 223. The first pin 2141 corresponds to the first socket hole 110, the second pin 2142 corresponds to the second socket hole 120, the third pin 2241 corresponds to the third socket hole 130, and the fourth pin 2242 corresponds to the fourth socket hole 140. The detection module 320 is electrically connected to the second socket hole 120 and the third socket hole 130, and the detection module 320 detects a resistance value between the second socket hole 120 and the third socket hole 130. The determination module 330 further determines, according to the resistance value detected by the detection module 320 and the resistance value of the second test resistor 380, whether the first substrate 211 and the second substrate 221 are successfully connected to the main machine 100.

Referring to FIG. 15, FIG. 15 is a schematic diagram of a circuit structure of a detector according to the fourth embodiment of the present application. The detector 300 provided in this embodiment is basically the same as the detector 300 provided in the first embodiment, except that in this embodiment, the detector 300 further comprises an integrated chip 390. One terminal of the integrated chip 390 is electrically connected to the first connector 214, and the other terminal of the integrated chip 390 is electrically connected to the second connector 224. The detection module 320 is electrically connected to an socket hole of the main machine 100, and the determination module 330 determines, according to whether the detection module 320 can read content of the integrated chip 390, whether the first substrate 211 and the second substrate 221 are successfully connected to the main machine.

Specifically, the first connector 214 comprises a first pin 2141 and a second pin 2142. The second connector 224 comprises a third pin 2241 and a fourth pin 2242. The main machine 100 comprises a first socket hole 110, a second socket hole 120, a third socket hole 130, and a fourth socket hole 140. The first substrate 211 is electrically connected to the first pin 2141 with the first wire 213, the integrated chip 390 is separately electrically connected to the second pin 2142 and the third pin 2241, and the second substrate 221 is electrically connected to the fourth pin 2242 with the second wire 223. The first pin 2141 corresponds to the first socket hole 110, the second pin 2142 corresponds to the second socket hole 120, the third pin 2241 corresponds to the third socket hole 130, and the fourth pin 2242 corresponds to the fourth socket hole 140. The detection module 320 is electrically connected to the second socket hole 120 and the third socket hole 130, and the determination module 330 determines, according to whether the detection module 320 can read the content of the integrated chip 390, whether the first substrate 211 and the second substrate 221 are successfully connected to the main machine 100.

Further, the integrated chip 390 stores a manufacturing date and an expiration date of the first electrode pad 210 and the second electrode pad 220, and when the detection module 320 reads the manufacturing date and the expiration date of the first electrode pad 210 and the second electrode pad 220 from the integrated chip 390, the determination module 330 further determines, according to a current date, the manufacturing date, and the expiration date, whether the first electrode pad 210 and the second electrode pad 220 have expired.

The embodiments of the disclosure have been described in detail above, and specific examples are used herein to explain the principles and implementation of the disclosure. The above description of the embodiments is only used to facilitate understanding of the invention. The scope of the invention is defined by the appended clams. sum up, the content of this specification should not be construed as limiting the disclosure.

## Claims

1. A defibrillator, comprising: a main machine, a first electrode pad, a second electrode pad, and a detector, the detector is electrically connected to the first electrode pad and the second electrode pad, wherein a contact resistance between the first electrode pad and the main machine is equivalent to that of a first resistor R1, a contact resistance between the second electrode pad and the main machine is equivalent to that of a second resistor R2, a capacitance between the first electrode pad and the second electrode pad is equivalent to that of a capacitor C, one terminal of the capacitor C is connected to the first resistor R1, and the other terminal of the capacitor C is connected to the second resistor R2; the detector comprises a signal generation module, a detection module, and a determination module, the signal generation module is configured to generate a test signal, the detection module is configured to detect a detection signal on the capacitor C generated after the test signal passes through the first electrode pad and the second electrode pad, and the determination module is configured to determine, according to the detection signal, whether the first electrode pad and the second electrode pad are successfully connected to the main machine and further determine whether the first electrode pad and the second electrode pad are in the normal state.

2. The defibrillator of claim 1, **characterized in that** the determination module is configured to determine that the first electrode pad and the second electrode pad are successfully connected to the main machine, when the detection module receives the detection signal; and
the determination module is configured to determine that the first electrode pad and the second electrode pad are not successfully connected to the main machine, when the detection module fails to detect the detection signal.

3. The defibrillator of claim 1, **characterized in that** the determination module is configured to determine, according to a magnitude of a resistance value in the detection signal detected by the detection module, whether the first electrode pad and the second electrode pad are in the normal state.

4. The defibrillator of claim 1, **characterized in that** the signal generation module comprises a first terminal and a second terminal, the first terminal is electrically connected to the first electrode pad, the second terminal is electrically connected to the second electrode pad, and the test signal is outputted to the first electrode pad and the second electrode pad via the first terminal and the second terminal.

5. The defibrillator of claim 1, **characterized in that** the detection signal is an analogue signal, the detector further comprises a sampling module, and the sampling module is configured to sample the detection signal; and
the determination module is configured to determine, according to the sampled detection signal, whether the first connector and the second connector are successfully connected to the main machine and further determine whether the first electrode pad and the second electrode pad are in the normal state;
the sampled detection signal is a digital signal.

6. The defibrillator of any one of claims 1 to 5, **characterized in that** the test signal comprises a first test sub-signal and a second test sub-signal, and the detection signal comprises a first detection sub-signal and a second detection sub-signal;
a corresponding detection signal is the first detection sub-signal when the first test sub-signal is loaded onto the first substrate and the second substrate; and
a corresponding detection signal is the second detection sub-signal when the second test sub-signal is loaded onto the first substrate and the second substrate; and
the determination module is configured to determine, according to a difference between the first detection sub-signal and the second detection sub-signal, whether the first connector and the second connector are successfully connected to the main machine and further determine whether the first electrode pad and the second electrode pad are in the normal state.

7. The defibrillator of claim 6, **characterized in that** the detector further comprises a time sequence control module, the time sequence control module is configured to control the first test sub-signal to be loaded onto the first electrode pad and the second electrode pad before the second test sub-signal, and a frequency of the second test sub-signal is different from that of the first test sub-signal.

8. The defibrillator of claim 6 or 7, **characterized in that** the detector further comprises a time sequence control module, the time sequence control module is configured to control the first test sub-signal to be loaded onto the first electrode pad and the second electrode pad before the second test sub-signal, and an amplitude value of the second test sub-signal is greater than that of the first test sub-signal.

9. The defibrillator of any one of claims 1 to 8, **characterized in that** the detector further comprises a first test resistor, one terminal of the first test resistor is electrically connected to the first substrate, and another terminal of the first test resistor is electrically connected to the second substrate, one terminal of the detection module is electrically connected to a node between the first test resistor and the first substrate, and another terminal of the detection module is electrically connected to a node between the first test resistor and the second substrate.

10. The defibrillator of any one of claims 1 to 8, **characterized in that** the detector further comprises a second test resistor, one terminal of the second test resistor is electrically connected to the first connector, and another terminal of the second test resistor is electrically connected to the second connector, the detection module is electrically connected to socket holes of the main machine, and the determination module is configured to determine, according to a resistance value of the second test resistor detected by the detection module and a resistance value of the second test resistor, whether the first electrode pad and the second electrode pad are successfully connected to the main machine.

11. The defibrillator of any one of claims 1 to 8, **characterized in that** the first electrode pad and the second electrode pad are inserted into socket holes in the main machine through plugs, the detector further comprises an integrated chip, one terminal of the integrated chip is electrically connected to a plug of the first electrode pad, another terminal of the integrated chip is electrically connected to a plug of the second electrode pad, the detection module is electrically connected to socket holes of the main machine, and the determination module determines, according to whether the detection module can read content of the integrated chip, whether the first electrode pad and the second electrode pad are successfully connected to the main machine.

12. The defibrillator of claim 11, **characterized in that** the first electrode pad comprises a first substrate, a first wire, and a first connector, the first connector comprises a first pin and a second pin, the second electrode pad comprises a second substrate, a second wire, and a second connector, the second connector comprises a third pin and a fourth pin, the socket holes comprises a first socket hole, a second socket hole, a third socket hole, and a fourth socket hole, the first substrate is electrically connected to the first pin with the first wire, the integrated chip is electrically connected to the second pin and the third pin, the second substrate is electrically connected to the fourth pin with the second wire, the first pin is correspondingly inserted into the first socket hole, the second pin is correspondingly inserted into the second socket hole, the third pin is correspondingly inserted into the third socket hole, the fourth pin is correspondingly inserted into the fourth socket hole, and the detection module is electrically connected to the second socket hole and the third socket hole.

13. The defibrillator of claim 12, **characterized in that** the integrated chip is configured to store a manufacturing date and an expiration date of the first electrode pad and the second electrode pad, and when the detection module reads the manufacturing date and the expiration date of the first electrode pad and the second electrode pad from the integrated chip, the determination module is further configured to determine, according to a current date, the manufacturing date and the expiration date, whether the first electrode pad and the second electrode pad have expired.

14. A set of electrode pads comprising: a first electrode pad, a second electrode pad, and a detector,
the first electrode pad comprising a first substrate, a first conductive adhesive, a first wire, and a first connector, the first conductive adhesive is defined on the first substrate, the first substrate is electrically connected to the first connector through the first wire;
the second electrode pad comprising a second substrate, a second conductive adhesive, a second wire, and a second connector, the second conductive adhesive is defined on the second substrate, the second substrate is electrically connected to the second connector through the second wire;
the first connector and the second connector are configured to be inserted into a main machine;
the detector is electrically connected to the first substrate and the second substrate;
wherein a contact resistance between the first electrode pad and the main machine is equivalent to that of a first resistor R1, a contact resistance between the second electrode pad and the main machine is equivalent to that of a second resistor R2, a capacitance between the first electrode pad and the second electrode pad is equivalent to that of a capacitor C, one terminal of the capacitor C is connected to the first resistor R1, and the other terminal of the capacitor C is connected to the second resistor R2; the detector comprises a signal generation module, a detection module, and a determination module, the signal generation module is configured to generate a test signal, the detection module is configured to detect a detection signal on the capacitor C generated after the test signal passes through the first electrode pad and the second electrode pad, and the determination module is configured to determine, according to the detection signal, whether the first electrode pad and the second electrode pad are successfully connected to the main machine and further determine whether the first electrode pad and the second electrode pad are in the normal state.

## Patentansprüche

1. Ein Defibrillator, bestehend aus einem Hauptgerät, einer ersten Elektrodenplatte, einer zweiten Elektrodenplatte und einem Detektor, wobeidieser elektrisch mit der ersten Elektrodenplatte und der zweiten Elektrodenplatte verbunden ist, ersten Widerstandes RI entspricht, ein Kontaktwiderstand zwischen der zweiten Elektrodenplatte und dem Hauptgerät dem eines zweiten Widerstandes R2 entspricht, eine Kapazität zwischen der ersten Elektrodenplatte und der zweiten Elektrodenplatte dem eines Kondensator C entspricht,ein Anschluss des Kondensators C ist mit dem ersten Widerstand RI verbunden ist, und der andere Anschluss des Kondensators C ist mit dem zweiten Widerstand R2 verbunden ist; der Detektor ein Signalerzeugungsmodul, ein Erfassungsmodul und ein Bestimmungsmodul umfasst, wobei das Signalerzeugungsmodul so konfiguriert ist, dass es ein Testsignal erzeugt, das Erfassungsmodul so konfiguriert ist, dass es ein Erfassungssignal an dem Kondensator C erfasst, das erzeugt wird, nachdem das Testsignal die erste Elektrodenplatte und die zweite Elektrodenplatte durchlaufen hat, und das Bestimmungsmodul so konfiguriert ist, dass es entsprechend dem Erfassungssignal bestimmt, ob die erste Elektrodenplatte und die zweite Elektrodenplatte korrekt an das Hauptgerät angeschlossen sind, und ferner bestimmt, ob sich die erste Elektrodenplatte und die zweite Elektrodenplatte im Normalzustand befinden.

2. Defibrillator nach Anspruch 1, **dadurch gekennzeichnet, dass** das Bestimmungsmodul so konfiguriert ist, dass es bestimmt, dass die erste Elektrodenplatte und die zweite Elektrodenplatte korrekt an das Hauptgerät angeschlossen sind, wenn das Erfassungsmodul das Erfassungssignal empfängt; und
das Bestimmungsmodul konfiguriert ist, um zu bestimmen, dass die erste Elektrodenplatte und die zweite Elektrodenplatte nicht korrekt an das Hauptgerät angeschlossen sind, wenn das Erfassungsmodul das Erfassungssignal nicht empfängt.

3. Defibrillator nach Anspruch 1, **dadurch gekennzeichnet, dass** das Bestimmungsmodul so konfiguriert, dass es anhand der Größe eines Widerstandswertes in dem vom Erfassungsmodul erkannten Erfassungssignal bestimmt, ob sich erste Elektrodenplatte und die zweite Elektrodenplatte im Normalzustand befinden.

4. Defibrillator nach Anspruch 1, **dadurch gekennzeichnet, dass** das Signalerzeugungsmodul einen ersten und einen zweiten Anschluss aufweist, wobei der erste Anschluss elektrisch mit der ersten Elektrodenplatte verbunden ist, der zweite Anschluss elektrisch mit der zweiten Elektrodenplatte verbunden ist, und das Testsignal über den ersten Anschluss und den zweiten Anschluss an die erste Elektrodenplatte und die zweite Elektrodenplatte ausgegeben wird.

5. Defibrillator nach Anspruch 1, **dadurch gekennzeichnet, dass** das Erfassungssignal ein analoges Signal ist, der Detektor ferner ein Probennahme-Modul umfasst, das so konfiguriert ist, dass es das Erfassungssignal abtastet; und
dass das Bestimmungsmodul so konfiguriert ist, dass es auf der Grundlage der abgetasteten Erfassungssignal feststellt, ob der erste Steckverbinder und der zweite Steckverbinder korrekt an das Hauptgerät angeschlossen sind, und ob sich das erste Elektrodenplatte und das zweite Elektrodenplatte im Normalzustand befinden;
das abgetastete Erfassungssignal ist ein digitales Signal.

6. Defibrillator nach einem der Ansprüche 1 bis 5, **dadurch gekennzeichnet, dass** das Testsignal ein erstes Test-Teilsignal und ein zweites Test-Teilsignal beinhaltet und das Erfassungssignal ein erstes Erfassungs-Teilsignal und ein zweites Erfassungs-Teilsignal beinhaltet;
ein entsprechendes Erfassungssignal das erste Erfassungs-Teilsignal ist, wenn das erste Test-Teilsignal auf das erste Substrat und das zweite Substrat geladen wird; und
ein entsprechendes Erfassungssignal das zweite Erfassungs-Teilsignal ist, wenn das zweite Test Teilsignal auf das erste Substrat und das zweite Substrat geladen wird; und
das Bestimmungsmodul ist konfiguriert, um gemäß einer Differenz zwischen dem ersten ErfassungsTeilsignal und dem zweiten Erfassungs-Teilsignal zu bestimmen, ob der erste Steckverbinder und der zweite Steckverbinder korrekt an das Hauptgerät angeschlossen sind und stellt ferner fest, ob sich das erste Elektrodenplatte und das zweite Elektrodenplatte im Normalzustand befinden.

7. Defibrillator nach Anspruch 6, **dadurch gekennzeichnet, dass** der Detektor ferner ein Modul zur Steuerung der Zeitabfolge umfasst, das so konfiguriert ist, dass es das erste Test-Teilsignal so steuert, dass es vor dem zweiten Test-Teilsignal auf das erste Elektrodenplatte und das zweite Elektrodenplatte geladen wird, und eine Frequenz des zweiten Test-Teilsignals sich von der des ersten Test-Teilsignals unterscheidet.

8. Defibrillator nach Anspruch 6 oder 7, **dadurch gekennzeichnet, dass** der Detektor ferner ein Modul zur Steuerung der Zeitabfolge umfasst, das so konfiguriert ist, dass es das erste Test-Teilsignal so steuert, dass es vor dem zweiten Test-Teilsignal auf das erste Elektrodenplatte und die zweite Elektrodenplatte geladen wird, und ein Amplitudenwert des zweiten Test-Teilsignals größer ist als der des ersten Test-Teilsignals.

9. Defibrillator nach einem der Ansprüche 1 bis 8, **dadurch gekennzeichnet, dass** der Detektor ferner einen ersten Testwiderstand umfasst, wobei ein Anschluss des ersten Testwiderstands elektrisch mit dem ersten Substrat verbunden ist und ein anderer Anschluss des ersten Testwiderstands mit dem zweiten Substrat verbunden ist, ein Anschluss des Erfassungsmoduls elektrisch mit einem Knoten zwischen dem ersten Testwiderstand und dem ersten Substrat verbunden ist und ein anderer Anschluss des Erfassungsmoduls elektrisch mit einem Knoten zwischen dem ersten Testwiderstand und dem zweiten Substrat verbunden ist.

10. Defibrillator nach einem der Ansprüche 1 bis 8, **dadurch gekennzeichnet, dass** der Detektor ferner einen zweiten Testwiderstand umfasst, wobei ein Anschluss des zweiten Testwiderstandes elektrisch mit dem ersten Steckverbinder und ein anderer Anschluss des zweiten Testwiderstandes elektrisch mit dem zweiten Steckverbinder verbunden ist, das Erfassungsmodul elektrisch mit Buchsen des Hauptgeräts verbunden ist und das Bestimmungsmodul ist so konfiguriert, dass es in Abhängigkeit von einem vom Erfassungsmodul erfassten Widerstandswert des zweiten Testwiderstands und einem Widerstandswert des zweiten Testwiderstands feststellt, ob das erste und das zweite Elektrodenplatte korrekt an das Hauptgerät angeschlossen sind.

11. Defibrillator nach einem der Ansprüche 1 bis 8, **dadurch gekennzeichnet, dass** die erste und die zweite Elektrodenplatte mittels Steckverbinder in Buchsen in dem Hauptgerät eingesetzt werden, der Detektor ferner einen integrierten Chip umfasst, ein Anschluss des integrierten Chips elektrisch mit einem Steckverbinder der ersten Elektrodenplatte verbunden ist, ein anderer Anschluss des integrierten Chips elektrisch mit einem Steckverbinder der zweiten Elektrodenplatte verbunden ist, das Erfassungsmodul elektrisch mittels Buchsen des Hauptgeräts verbunden ist, und das Bestimmungsmodul, je nachdem, ob das Erfassungsmodul den Inhalt des integrierten Chips lesen kann, feststellt, ob die erste und die zweite Elektrodenplatte korrekt an das Hauptgerät angeschlossen sind.

12. Defibrillator nach Anspruch 11, **dadurch gekennzeichnet, dass** die erste Elektrodenplatte ein erstes Substrat, einen ersten Draht und einen ersten Steckverbinder aufweist, wobei der erste Steckverbinder einen ersten Stift und einen zweiten Stift umfasst, die zweite Elektrodenplatte ein zweites Substrat, einen zweiten Draht und einen zweiten Steckverbinder aufweist, wobei der zweite Steckverbinder einen dritten Stift und einen vierten Stift umfasst, die Buchsen eine erste Buchse, eine zweite Buchse, eine dritte Buchse und eine vierte Buchse aufweisen, das erste Substrat elektrisch mit dem ersten Stift mit dem ersten Draht verbunden ist, der integrierte Chip elektrisch mit dem zweiten Stift und dem dritten Stift verbunden ist, das zweite Substrat elektrisch mit dem vierten Stift mit dem zweiten Draht verbunden ist, der erste Stift entsprechend in die erste Buchse eingesetzt wird, der zweite Stift entsprechend in die zweite Buchse eingesetzt wird, der dritte Stift entsprechend in die dritte Buchse eingesetzt wird, der vierte Stift entsprechend in die vierte Buchse eingesetzt wird, und das Erfassungsmodul elektrisch mit der zweiten Buchse und der dritten Buchse verbunden ist.

13. Defibrillator nach Anspruch 12, **dadurch gekennzeichnet, dass** der integrierte Chip so konfiguriert ist, dass er ein Herstellungsdatum und ein Verfallsdatum des ersten und der zweiten Elektrodenplatte speichert, und wenn das Erfassungsmodul das Herstellungsdatum und das Verfallsdatum des ersten und der zweiten Elektrodenplatte von dem integrierten Chip liest, ist das Bestimmungsmodul ferner so konfiguriert, dass es gemäß einem aktuellen Datum, dem Herstellungsdatum und dem Verfallsdatum feststellt, ob das erste und das zweite Elektrodenplatte verfallen sind.

14. Ein Set von Elektrodenplatten bestehend aus einer ersten Elektrodenplatte, einer zweiten Elektrodenplatte und einem Detektor,
wobei die erste Elektrodenplatte aus einem ersten Substrat und einem ersten leitfähigen Klebstoff, einem ersten Draht und einem ersten Steckverbinder besteht, wobei der erste leitfähige Klebstoff auf dem ersten Substrat aufgetragen wird und das erste Substrat über den ersten Draht elektrisch mit dem ersten Steckverbinder verbunden ist;
die zweite Elektrodenplatte ein zweites Substrat, einen zweiten leitfähigen Klebstoff, einen zweiten Draht und einen zweiten Steckverbinder umfasst, wobei der zweite leitfähige Klebstoff auf dem zweiten Substrat aufgetragen wird und das zweite Substrat über den zweiten Draht elektrisch mit dem zweiten Steckverbinder verbunden ist;
der erste und der zweite Steckverbinder so konfiguriert sind, dass sie in ein Hauptgerät eingesetzt werden können;
Detektor elektrisch mit dem ersten und dem zweiten Substrat verbunden ist;
wobei ein Kontaktwiderstand zwischen der ersten Elektrodenplatte und dem Hauptgerät dem eines ersten Widerstandes RI entspricht, ein Kontaktwiderstand zwischen der zweiten Elektrodenplatte und dem Hauptgerät dem eines zweiten Widerstand R2 entspricht, eine Kapazität zwischen der ersten und der zweiten Elektrodenplatte der eines Kompensators C entspricht, ein Anschluss des Kondensators C mit dem ersten Widerstand RI verbunden ist, und der andere Anschluss des Kondensators C mit dem zweiten Widerstand R2 verbunden ist; der Detektor umfasst ein Signalerzeugungsmodul, ein Erfassungsmodul und ein Bestimmungsmodul, das Signalerzeugungsmodul ist mit dem ersten Widerstand RI verbunden und so konfiguriert, dass es ein Testsignal erzeugt, das Erfassungsmodul ist so konfiguriert, dass es ein Erfassungssignal an dem Kondensator C erkennt, das erzeugt wird, nachdem das Testsignal die erste und die zweite Elektrodenplatte durchlaufen hat, und das Bestimmungsmodul ist so konfiguriert, dass es gemäß dem Erfassungssignal feststellt, ob die erste und die zweite Elektrodenplatte korrekt an das Hauptgerät angeschlossen sind, und ferner bestimmt, ob sich die erste und die zweite Elektrodenplatte im Normalzustand befinden.

## Revendications

1. Défibrillateur, comprenant : une machine principale, un premier tampon d'électrode, un second tampon d'électrode, et un détecteur, le détecteur est électriquement relié au premier tampon d'électrode et au second tampon d'électrode, où une résistance de contact entre le premier tampon d'électrode et la machine principale est équivalente à celle d'une première résistance R1, une résistance de contact entre le second tampon d'électrode et la machine principale est équivalente à celle d'une seconde résistance R2, une capacité entre le premier tampon d'électrode et le second tampon d'électrode est équivalente à celle d'un condensateur C, une première borne du condensateur C est reliée à la première résistance R1, et l'autre borne du condensateur C est reliée à la seconde résistance R2 ; le détecteur comprend un module de génération de signal, un module de détection, et un module de détermination, le module de génération de signal est configuré pour générer un signal d'essai, le module de détection est configuré pour détecter un signal de détection sur le condensateur C généré après que le signal d'essai est passé par le premier tampon d'électrode et le second tampon d'électrode, et le module de détermination est configuré pour déterminer, en fonction du signal de détection, si le premier tampon d'électrode et le second tampon d'électrode sont reliés avec succès à la machine principale et déterminer en outre si le premier tampon d'électrode et le second tampon d'électrode se trouvent dans l'état normal.

2. Défibrillateur selon la revendication 1, **caractérisé en ce que** le module de détermination est configuré pour déterminer que le premier tampon d'électrode et le second tampon d'électrode sont reliés avec succès à la machine principale, lorsque le module de détection reçoit le signal de détection ; et
le module de détermination est configuré pour déterminer que le premier tampon d'électrode et le second tampon d'électrode ne sont pas reliés avec succès à la machine principale, lorsque le module de détection ne parvient pas à détecter le signal de détection.

3. Défibrillateur selon la revendication 1, **caractérisé en ce que** le module de détermination est configuré pour déterminer, en fonction d'une ampleur d'une valeur ohmique dans le signal de détection détectée par le module de détection, si le premier tampon d'électrode et le second tampon d'électrode se trouvent dans l'état normal.

4. Défibrillateur selon la revendication 1, **caractérisé en ce que** le module de génération de signal comprend une première borne et une seconde borne, la première borne est électriquement reliée au premier tampon d'électrode, la seconde borne est électriquement reliée au second tampon d'électrode, et le signal d'essai est fourni au premier tampon d'électrode et au second tampon d'électrode via la première borne et la seconde borne.

5. Défibrillateur selon la revendication 1, **caractérisé en ce que** le signal de détection est un signal analogique, le détecteur comprend en outre un module d'échantillonnage, et le module d'échantillonnage est configuré pour échantillonner le signal de détection ; et
le module de détermination est configuré pour déterminer, en fonction du signal de détection échantillonné, si le premier connecteur et le second connecteur sont reliés avec succès à la machine principale et déterminer en outre si le premier tampon d'électrode et le second tampon d'électrode se trouvent dans l'état normal ;
le signal de détection échantillonné est un signal numérique.

6. Défibrillateur selon l'une quelconque des revendications 1 à 5, **caractérisé en ce que** le signal d'essai comprend un premier sous-signal d'essai et un second sous-signal d'essai, et le signal de détection comprend un premier sous-signal de détection et un second sous-signal de détection ;
un signal de détection correspondant est le premier sous-signal de détection lorsque le premier sous-signal d'essai est chargé sur le premier substrat et le second substrat ; et
un signal de détection correspondant est le second sous-signal de détection lorsque le second sous-signal d'essai est chargé sur le premier substrat et le second substrat ; et
le module de détermination est configuré pour déterminer, en fonction d'une différence entre le premier sous-signal de détection et le second sous-signal de détection, si le premier connecteur et le second connecteur sont reliés avec succès à la machine principale et déterminer en outre si le premier tampon d'électrode et le second tampon d'électrode se trouvent dans l'état normal.

7. Défibrillateur selon la revendication 6, **caractérisé en ce que** le détecteur comprend en outre un module de commande de séquence temporelle, le module de commande de séquence temporelle est configuré pour commander le premier sous-signal d'essai devant être chargé sur le premier tampon d'électrode et le second tampon d'électrode avant le second sous-signal d'essai, et une fréquence du second sous-signal d'essai est différente de celle du premier sous-signal d'essai.

8. Défibrillateur selon la revendication 6 ou 7, **caractérisé en ce que** le détecteur comprend en outre un module de commande de séquence temporelle, le module de commande de séquence temporelle est configuré pour commander le premier sous-signal d'essai devant être chargé sur le premier tampon d'électrode et le second tampon d'électrode avant le second sous-signal d'essai, et une valeur d'amplitude du second sous-signal d'essai est différente de celle du premier sous-signal d'essai.

9. Défibrillateur selon l'une quelconque des revendications 1 à 8, **caractérisé en ce que** le détecteur comprend en outre une première résistance d'essai, une première borne de la première résistance d'essai est électriquement reliée au premier substrat, et l'autre borne de la première résistance d'essai est électriquement reliée au second substrat, une première borne du module de détection est électriquement reliée à un noeud entre la première résistance d'essai et le premier substrat, et l'autre borne du module de détection est électriquement reliée à un noeud entre la première résistance d'essai et le second substrat.

10. Défibrillateur selon l'une quelconque des revendications 1 à 8, **caractérisé en ce que** le détecteur comprend en outre une seconde résistance d'essai, une première borne de la seconde résistance d'essai est électriquement reliée au premier connecteur, et l'autre borne de la seconde résistance d'essai est électriquement reliée au second connecteur, le module de détection est électriquement relié à des trous de douille de la machine principale, et le module de détermination est configuré pour déterminer, en fonction d'une valeur ohmique de la seconde résistance d'essai détectée par le module de détection et d'une valeur ohmique de la seconde résistance d'essai, si le premier tampon d'électrode et le second tampon d'électrode sont reliés avec succès à la machine principale.

11. Défibrillateur selon l'une quelconque des revendications 1 à 8, **caractérisé en ce que** le premier tampon d'électrode et le second tampon d'électrode sont insérés dans les trous de douille dans la machine principale via des fiches, le détecteur comprend en outre une puce intégrée, une première borne de la puce intégrée est électriquement reliée à une fiche du premier tampon d'électrode, l'autre borne de la puce intégrée est électriquement reliée à une fiche du second tampon d'électrode, le module de détection est électriquement relié à des trous de douille de la machine principale, et le module de détermination détermine, selon que le module de détection peut lire le contenu de la puce intégrée ou non, si le premier tampon d'électrode et le second tampon d'électrode sont reliés avec succès à la machine principale.

12. Défibrillateur selon la revendication 11, **caractérisé en ce que** le premier tampon d'électrode comprend un premier substrat, un premier fil, et un premier connecteur, le premier connecteur comprend une première broche et une deuxième broche, le second tampon d'électrode comprend un second substrat, un second fil, et un second connecteur, le second connecteur comprend une troisième broche et une quatrième broche, les trous de douille comprennent un premier trou de douille, un deuxième trou de douille, un troisième trou de douille et un quatrième trou de douille, le premier substrat est électriquement relié à la première broche avec le premier fil, la puce intégrée est électriquement reliée à la deuxième broche et à la troisième broche, le second substrat est électriquement relié à la quatrième broche avec le second fil, la première broche est insérée en correspondance dans le premier trou de douille, la deuxième broche est insérée en correspondance dans le deuxième trou de douille, la troisième broche est insérée en correspondance dans le troisième trou de douille, la quatrième broche est insérée en correspondance dans le quatrième trou de douille, et le module de détection est électriquement relié au deuxième trou de douille et au troisième trou de douille.

13. Défibrillateur selon la revendication 12, **caractérisé en ce que** la puce intégrée est configurée pour mémoriser une date de fabrication et une date de péremption du premier tampon d'électrode et du second tampon d'électrode, et lorsque le module de détection lit la date de fabrication et la date de péremption du premier tampon d'électrode et du second tampon d'électrode sur la puce intégrée, le module de détermination est en outre configuré pour déterminer, en fonction d'une date actuelle, de la date de fabrication et de la date de péremption, si le premier tampon d'électrode et le second tampon d'électrode sont périmés.

14. Ensemble de tampons d'électrode comprenant : un premier tampon d'électrode, un second tampon d'électrode, et un détecteur,
le premier tampon d'électrode comprenant un premier substrat, un premier adhésif conducteur, un premier fil, et un premier connecteur, le premier adhésif conducteur est délimité sur le premier substrat, le premier substrat est électriquement relié au premier connecteur via le premier fil ;
le second tampon d'électrode comprenant un second substrat, un second adhésif conducteur, un second fil, et un second connecteur, le second adhésif conducteur est délimité sur le second substrat, le second substrat est électriquement relié au second connecteur via le second fil;
le premier connecteur et le second connecteur sont configurés pour être insérés dans une machine principale ;
le détecteur est électriquement relié au premier substrat et au second substrat ;
où une résistance de contact entre le premier tampon d'électrode et la machine principale est équivalente à celle d'une première résistance R1, une résistance de contact entre le second tampon d'électrode et la machine principale est équivalente à celle d'une seconde résistance R2, une capacité entre le premier tampon d'électrode et le second tampon d'électrode est équivalente à celle d'un condensateur C, une première borne du condensateur C est reliée à la première résistance R1, et l'autre borne du condensateur C est reliée à la seconde résistance R2 ; le détecteur comprend un module de génération de signal, un module de détection, et un module de détermination, le module de génération de signal est configuré pour générer un signal d'essai, le module de détection est configuré pour détecter un signal de détection sur le condensateur C généré après que le signal d'essai est passé par le premier tampon d'électrode et le second tampon d'électrode, et le module de détermination est configuré pour déterminer, en fonction du signal de détection, si le premier tampon d'électrode et le second tampon d'électrode sont reliés avec succès à la machine principale et déterminer en outre si le premier tampon d'électrode et le second tampon d'électrode se trouvent dans l'état normal.
